(19) Europäisches Patentamt
European Patent Office
Office européen des brevets

(11) **EP 3 069 716 A1**

(12) **EUROPEAN PATENT APPLICATION**
published in accordance with Art. 153(4) EPC

(43) Date of publication:
**21.09.2016 Bulletin 2016/38**

(51) Int Cl.:
*A61K 9/70* (2006.01)   *A61P 3/10* (2006.01)
*A61P 3/04* (2006.01)

(21) Application number: **14861454.8**

(22) Date of filing: **30.10.2014**

(86) International application number:
**PCT/KR2014/010293**

(87) International publication number:
**WO 2015/072684 (21.05.2015 Gazette 2015/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.11.2013 KR 20130138040**

(71) Applicant: **Seoul Pharma. Co., Ltd.**
**Siheung-si, Gyeonggi-do 15093 (KR)**

(72) Inventors:
• **OH, Dong Hoon**
  **Siheung-si (KR)**
• **LEE, Jin Hoo**
  **Siheung-si (KR)**
• **LEE, So Yi**
  **281, Hyeomnyeok-ro (KR)**
• **KANG, Jun Hyuk**
  **Siheung-si (KR)**
• **LIM, Chae Yun**
  **Ansan-si (KR)**

• **JUNG, Kyung Tae**
  **210, Godeok-ro (KR)**
• **YEON, Kyu Jeong**
  **212, Samseong-ro (KR)**
• **PARK, Jin Gyu**
  **17, Gobong-ro 278beon-gil (KR)**
• **CHOE, Dong Woon**
  **Suwon-si**
  **Gyeonggi-do 16336 (KR)**
• **PARK, Mi Ran**
  **Ansan-si**
  **Gyeonggi-do 15307 (KR)**
• **SHIN, Jun Ho**
  **Ansan-si**
  **Gyeonggi-do 15473 (KR)**
• **KIM, Hyun Soo**
  **Siheung-si**
  **Gyeonggi-do 15002 (KR)**

(74) Representative: **D Young & Co LLP**
**120 Holborn**
**London EC1N 2DY (GB)**

(54) **ORALLY DISINTEGRATING POROUS FILM COMPRISING PHARMACOLOGICAL ACTIVE INGREDIENT AND METHOD FOR PREPARING SAME**

(57)    Disclosed are an orally disintegrating porous film and a method of preparing the same, wherein the orally disintegrating porous film includes a foaming agent, a foam stabilizer, a plasticizer, and a pharmacologically active ingredient. This orally disintegrating porous film possesses properties suitable for a film and also has micropores therein, thus significantly improving the drug dissolution rate, and can be easily prepared at low cost through a simple preparation process, thereby exhibiting superior processability.

[Fig.3]

Aripiprazole ODF dissolution rate **VS.** Control dissolution rate

Conditions) pH 4, 50rpm, 900ml, paddle method
A: Example 1 Porous aripiprazole ODF
B: Comparative Example 3 Non-porous aripiprazole ODF
R: Control (abilify Tablet)

EP 3 069 716 A1

**Description**

**Technical Field**

The present invention relates to an orally disintegrating porous film, which includes a foaming agent, a foam stabilizer, a plasticizer, and a pharmacologically active ingredient, and a method of preparing the same.

**Background Art**

Oral dosage forms may include a variety of orally disintegrating formulations, such as tablets, chewable tablets, sublingual tablets, capsules, liquids, etc. Among them, typical tablets or capsules are problematic because they are not easy to apply to patients who have a difficulty in taking drugs, and liquids suffer from low stability and inaccurate administration dose. Hence, the need for novel formulations that may be easily taken arises.

Recently, dosage forms having new drug delivery systems have come to the fore through a variety of studies. In this regard, an orally disintegrating tablet (ODT), in which a solid is developed in an orally disintegrating form, is exemplary. However, orally disintegrating tablets are problematic because not all drugs are uniformly disintegrated within a short time and the need for taking water again is common.

With the goal of solving the above problems, a newly proposed dosage form is an orally disintegrating film formulation that is frequently mentioned these days. Such an orally disintegrating film formulation provides some advantages, unlike conventional solids, liquids, and orally disintegrating tablets. Since the orally disintegrating film formulation may be taken without water, it is very useful for children, handicapped persons, bedridden patients, and busy modern people, as well as old people who have a difficulty in taking tablets or capsules, and the drug disintegration thereof is evaluated to be more advanced than any other dosage forms. Particularly in the case where drugs are absorbed into the oral mucous membrane, liver first-pass effects may be avoided, and thus, the orally disintegrating film is advantageous because it may be applied to drugs that are extensively metabolized by the liver, among the drugs absorbed via the alimentary canal. Thus, numerous attempts have been made to prepare orally disintegrating film formulations of various techniques for the properties of films and patient compliance. Despite the above advantages, such a film has defects, such as low initial dissolution and low dissolution rate of a drug, compared to typical tablets or orally disintegrating tablets, and thus it is difficult to develop generic drugs having bioequivalence and incrementally modified drugs.

As for the orally disintegrating films, a film forming agent may include a polymer, such as hydroxypropyl methylcellulose (hereinafter HPMC), pullulan, polyvinylacetate (hereinafter PVA), polyethylene oxide (hereinafter PEO), gelatin, alginic acid, etc., which may exhibit good film forming capability and is mainly used in an amount of 10 to 90 wt% based on the total solid content of the orally disintegrating film. As the amount of the added polymer is increased, the drug disintegration rate or dissolution rate is decreased. Furthermore, in the case where a pharmaceutically active ingredient is poorly water soluble, a drastic reduction in the dissolution rate is regarded as a large obstacle in the development of formulations.

Conventional methods of increasing the solubility of poorly water soluble drugs are known to include chemical modification and physical modification. A typical method for chemical modification may include salt addition and aqueous prodrug approach, and a typical method for physical modification may include changes in a particle size, changes in crystal form, formation of a crystalline polymorph, formation of a composite using a surfactant or cyclodextrin, and dispersion of a drug using a dispersant. The chemical modification method is difficult to apply depending on the type of the pharmaceutically active ingredient, and is thus difficult to generalize, and the inherent bitter taste of the active ingredient may be more evident with an increase in solubility. The physical method may be easily applied to tablets or capsules, but is difficult to apply to film formulations in practice because the suspension or solution is prepared and then dried during the preparation process. In particular, a solid dispersion method, which is frequently applied, is difficult to use to prepare a film, and thus limitations are imposed on the production of film formulations. Also, US patent application No. 2012/0149713 discloses the use of a solubilizer to increase the dissolution rate of the orally disintegrating film of aripiprazole, but this method typically cannot be applied to increase the dissolution rate of an orally disintegrating film since the method uses a specific crystal form.

In the typical formation of the orally disintegrating film, an orally disintegrating porous film is configured such that a thin film tissue is imparted with porosity, whereby the physical properties of the film deteriorate and the strength of the film remarkably decrease. Taking into consideration the quality of products, a non-porous film is used. Specifically, in order to defoam the film solution, the film solution is allowed to stand for a long period of time, or the film solution is stirred at a very low rpm, which may be disclosed in US Patent No. 7,425,292, and Korean Patent Nos. 10-1303479 and 10-1188594 regarding the method of preparing the orally disintegrating film including the defoaming step.

Some studies have been conducted on the preparation of an oral film having pores, such as Korean Patent Application Publication No. 10-2013-0003511 and Korean Patent No. 10-0937625. However, Korean Patent Application Publication No. 10-2013-0003511 is problematic because pores are not formed in the film solution itself, but a rapid disintegrating template having micropores is manufactured and a drug is separately applied thereon and dried, yielding a rapidly orally disintegrating film containing a pharmaceutically active ingredient, undesirably resulting in very low industrial applicability.

Also, Korean Patent No. 10-0937625 discloses a soluble web porous film and a method of preparing the same, wherein a soluble web porous film having a fiber-layered mesh structure is obtained by spinning a polymer for forming a fiber, which undesirably complicates the preparation process.

As for orally disintegrating films, which are receiving attention these days, film formulations and methods of preparing the same in which the film formulation is stably formed through a simple process and the dissolution rate of poorly water soluble drugs may be drastically increased have not yet been known, and research leading thereto is still required.

### Disclosure

### Technical Problem

Culminating in the present invention, thorough research into improvements in drug dissolution rates of orally disintegrating films through a simple preparation process, carried out by the present inventors, resulted in the finding that when a foaming agent, a foam stabilizer, and a plasticizer are appropriately used, the resulting orally disintegrating porous film may significantly increase the drug dissolution rate while preventing brittleness that may occur in porous films.

Accordingly, an object of the present invention is to provide an orally disintegrating porous film comprising a foaming agent, a foam stabilizer, a plasticizer, and a pharmacologically active ingredient and a method of preparing the same.

### Technical Solution

In order to accomplish the above object, the present invention provides an orally disintegrating porous film, comprising a foaming agent, a foam stabilizer, a plasticizer, and a pharmacologically active ingredient.

In addition, the present invention provides a method of preparing an orally disintegrating porous film, comprising:

(a) dissolving a foaming agent, a plasticizer and an excipient in a solvent to prepare a foamed solution; (b) homogenizing the solution at 4,000 rpm to 15,000 rpm using a homogenizer to prepare a homogenous solution; (c) adding a foam stabilizer to the homogenous solution and stirring the homogenous solution at 500 rpm to 1,500 rpm using a stirrer to prepare a foam-stabilized film solution; and (d) drying the film solution to mold a film, and also provides an orally disintegrating porous film prepared by the above method.

### Advantageous Effects

According to the present invention, the orally disintegrating porous film has properties suitable for a film and also has micropores therein, thus remarkably improving a drug dissolution rate. Furthermore, the orally disintegrating porous film can be easily prepared at low cost through a simple preparation process, thus exhibiting outstanding processability.

### Description of Drawings

FIG. 1 illustrates the results of microscopic observation at 1200x magnification for the cross-section, thickness and surface of the film of Comparative Example 3;

FIG. 2 illustrates the results of microscopic observation at 1200x magnification for the cross-section, thickness and surface of the film of Example 1; and

FIG. 3 illustrates the results of measurement of aripiprazole dissolution rate of the films of Comparative Example 3 and Example 1 and of a comparison thereof with a control (A: Example 1, B: Comparative Example 3, R: Control, ODF: Orally Disintegrating Film).

### Best Mode

The present invention provides an orally disintegrating porous film, including a foaming agent, a foam stabilizer, a plasticizer, and a pharmacologically active ingredient.

The orally disintegrating porous film according to the present invention has properties suitable for a film and also has micropores therein, thus significantly improving the drug dissolution rate, and can be easily prepared at low cost through a simple preparation process, thereby exhibiting superior processability.

In the present invention, a disintegrating porous film refers to a disintegrating film having micropores therein.

The foaming agent may be at least one selected from the group consisting of a surfactant, an animal-based foaming agent (casein, gelatin, etc.), a polymer foaming agent (a cellulose based polymer, a polyacrylate based polymer, etc.), and a mineral-based foaming agent (a dodecyl benzene based compound or an aluminum powder). Preferably the foaming agent is a surfactant. The addition of the surfactant facilitates the generation of microfoam in the preparation of a film solution.

The surfactant may be at least one selected from the group consisting of a nonionic surfactant, a cationic surfactant, an anionic surfactant, and an amphoteric surfactant, and may be at least one selected from the group consisting of glycerin fatty acid ester, sucrose fatty acid ester, lecithin, enzyme-treated lecithin, polysorbate, sorbitan fatty acid ester, and sucrose fatty acid ester.

In the orally disintegrating porous film according to the present invention, the foaming agent may be contained in an amount of 0.1 wt% to 10 wt%, and preferably 0.5 wt% to 10 wt%, based on the total weight of the orally disintegrating porous film. If the amount of the foaming agent exceeds 10 wt% based on the total weight of the orally disintegrating porous film, the amount of generated foam is increased but excessive foaming may deteriorate the formation of a film and the properties of the formed film. On the other hand, if the amount of the foaming agent is less than 0.5 wt%, the porous film intended in the present invention cannot be prepared.

The foam stabilizer may be a thickener, a gelling agent, an aqueous polymer, a non-aqueous polymer, or a combination thereof, and preferably is at least one selected from the group consisting of pullulan, hydroxypropyl methylcellulose, polyvinylacetate, polyethylene oxide, xanthan gum, guar gum, locust bean gum, starch and starch derivatives, pectin and pectin hydrolyzates, alginic acid and alginic acid hydrolyzates. A thickener, a gelling agent, an aqueous polymer, a non-aqueous polymer, or a combination thereof can stabilize the foam by forming the film and adjusting the viscosity of the film solution.

The foam stabilizer may further include at least one additive selected from the group consisting of alkyl cellulose, hydroxyalkyl cellulose, hydroxyalkyl alkylcellulose, carboxyalkyl cellulose, carboxyalkyl alkylcellulose, alkali metal salts of carboxyalkyl cellulose, carboxyalkyl cellulose ester, polyvinylalcohol, polyvinylpyrrolidone, polyalkylene glycol, polyalkylene oxide, carrageenan, alginic acid, alkali metal alginate, watersoluble chitosan, gluconic acid, polyaniline, cellulose acetate, polypyrrole, poloxamer, Pluronic F-127, phenylalanine-containing protein, lecithin, and Carbopol.

The foam stabilizer is preferably contained in an amount of 20 wt% to 90 wt% based on the total weight of the orally disintegrating porous film.

The plasticizer is used to supplement the strength of the orally disintegrating porous film, and may remarkably increase the flexibility of the film. The plasticizer preferably includes at least one selected from the group consisting of glycerin, glycerol oleate, medium chain fatty acid, polyethylene glycol, propylene glycol, propylene glycol monocaprylate, propylene glycol dicaprylate, saccharides, sugar alcohols, and triacetin.

The plasticizer is contained in an amount of 0.1 to 30 wt%, and preferably 0.5 to 20 wt%, based on 100 wt% of the foam stabilizer. If the amount of the plasticizer is small, the flexibility of the film may decrease. On the other hand, if the amount thereof is excessive, workability may decrease upon slitting or cutting.

According to the present invention, the orally disintegrating porous film may further optionally include a pH controller, an excipient, a sweetener, a flavor, a coloring agent, an oil, a humectant, a disintegrant, a lubricant, etc, which are typically used to prepare an orally disintegrating film, in addition to the foaming agent, the foam stabilizer, the plasticizer, and the pharmacologically active ingredient.

The excipient is a pharmaceutically acceptable excipient, and may include a lubricant, a disintegrant, a humectant, a buffer agent, a diluent, etc.

The pH controller may include potassium dihydrogen phosphate, sodium hydroxide, sodium bicarbonate, sodium phosphate, ammonium hydroxide, sodium stannate, triethanolamine, citric acid, hydrochloric acid, sodium citrate, and combinations thereof, and preferably includes potassium dihydrogen phosphate and/or sodium hydroxide. The pH controller is added in a sufficient amount, so that the pH of the film may be appropriately adjusted.

The sweetener preferably is at least one high-intensity sweetener selected from among aspartame, acesulfame salts, sucralose, saccharin salts, Neotame, cyclamate, thaumatin, Siraitia grosvenorii extract, and Glycyrrhiza uralensis extract, and more preferably is at least one high-intensity sweetener selected from among aspartame, sucralose, Neotame, and acesulfame salts.

The flavor may include a natural flavor, an artificial flavor, or a mixture thereof, without limitation, and preferably includes extracts from leaves, flowers and fruit of plants, vegetable oil, etc. Also, the artificial flavor may include an artificial synthetic fruit flavor such as lemon, orange, grape, lime, strawberry and the like, and an artificial synthetic fragrance such as vanilla, chocolate, coffee, cocoa, pine needles, ginseng, red ginseng, and citrus.

The coloring agent may include riboflavin, beta-carotene, anthocyan, carmine, indigo carmine, orange yellow S, quinoline yellow, indigotine lake, brilliant blue, and sunset yellow.

The oil may include safflower oil, castor oil, coconut oil, cottonseed oil, canola oil, herring oil, palm fruit oil, palm oil, peanut oil, soybean oil, rapeseed oil, linseed oil, rice bran oil, pine oil, sesame oil, sunflower seed oil, hardened safflower oil, and mixtures thereof.

The disintegrant may include starch, modified starch, methylcellulose, calcium carboxymethylcellulose, sodium carboxymethylcellulose, hydroxypropyl cellulose, microcrystalline cellulose, colloidal silicon dioxide, croscarmellose sodium, gelatinized starch, clay, cellulose, powdered cellulose, gelatinized starch, sodium alginate, alginic acid, guar gum, magnesium aluminum silicate, polacrilin potassium, or mixtures thereof.

The lubricant may include talc, stearic acid, magnesium stearate, colloidal silicon dioxide, sodium stearyl fumarate, glyceryl behenate, and glyceryl distearate.

According to the present invention, the orally disintegrating porous film has therein micropores, and the size of the micropores is 10 to 150, preferably 20 to 120, and more preferably 30 to 100. Also, the orally disintegrating porous film according to the present invention has therein a porosity of 8% to 40%, preferably 10% to 35%, and more preferably 15% to 30%. In the case where the orally disintegrating film has therein micropores having a size of greater than 150 and an excessively high porosity, the properties of the film may deteriorate. On the other hand, in the case where the size of the micropores is less than 10 and the porosity is excessively low, dissolution of the pharmacologically active ingredient in the film may be impeded.

The pharmacologically active ingredient, which is incorporated in the orally disintegrating porous film according to the present invention, may contain a drug without limitation so long as the drug may be prepared as an oral formulation, and preferably includes at least one selected from the group consisting of a therapeutic agent for diabetes, a therapeutic agent for insomnia, a therapeutic agent for urogenital organ, a therapeutic agent for obesity, an enzyme agent, an agent for peptic ulcer, an antitussive and an expectorant, a therapeutic agent for skin disease, an antiemetic agent, an antidepressant, an antihistamine agent, an antipyretic analgesic anti-inflammatory drug, a hormonal agent, a therapeutic agent for circulatory organ, a therapeutic agent for digestive organ, a cardiovascular agent, a psychotropic agent, a therapeutic agent for erectile dysfunction, a therapeutic agent for osteoporosis, a therapeutic agent for arthritis, a therapeutic agent for epilepsy, a muscle relaxant, a brain function-improving agent, a therapeutic agent for schizophrenia, an immunosuppressant, an antibiotic agent, an anticancer drug, an anticancer treatment adjuvant, a vaccine agent, a mouth cleaning agent, a therapeutic agent for anemia, a therapeutic agent for constipation, a vitamin, a nutrient, a lactic acid bacteria agent, a multi-symptom cold medicine, an animal drug, and health functional food.

Preferably the pharmacologically active ingredient is at least one selected from the group consisting of L-menthol, nicotine, benzocaine, ascorbic acid, phentermine hydrochloride, caffeine, baclofen, memantine hydrochloride, selegiline hydrochloride, nitroglycerin, simethicone, diethyl propion, lamotrigine, pemirolast potassium, voglibose, mazindol, tizanidine hydrochloride, diclofenac, ramipril, ambroxol hydrochloride, alprazolam, lansoprazole, levonorgestrel, metoclopramide hydrochloride, famotidine, topiramate, tylpyridinium chloro, phendimetrazine tartrate, triazolam, omeprazole, ranitidine hydrochloride, torasemide, olopatadine hydrochloride, doxylamine succinate, dexamethasone, oxybutynin hydrochloride, citalopram bromide, tarafenacin, tamsulosin, sildenafil citrate, sildenafil, tadalafil, udenafil, mirodenafil hydrochloride, vardenafil hydrochloride, dapoxetine, donepezil hydrochloride, escitalopram oxalate, aripiprazole, atomoxetine hydrochloride, olanzapine, risperidone, meclizine, ramosetron hydrochloride, granisetron, ondansetron hydrochloride, ondansetron, aprepitant, montelukast, montelukast sodium, loratadine, desloratadine, chloropheniramine, phenylephrine hydrochloride, diphenhydramine hydrochloride, pseudoephedrine hydrochloride, levocetirizine hydrochloride, cetirizine hydrochloride, dextromethorphan bromide, ketoprofen, ibuprofen, acetaminophen, rizatriptan, zolmitriptan, sumatriptan, meloxicam, zolpidem, doxazosin mesylate, cimetidine, fentanyl, desmopressin, buprenorphine, oxycodone hydrochloride, naloxone hydrochloride, tianeptine, ebastine, solifenacin succinate, nicergoline, glimepiride, mosapride, loperamide hydrochloride, fesoterodine fumarate, fexofenadine hydrochloride, olmesartan medoxomil, amlodipine besylate, pharmaceutically acceptable salts or free bases thereof, and mixtures thereof.

The pharmacologically active ingredient may be appropriately provided in the form of crystalline particles, as necessary, and the size thereof preferably falls in the range of 100 nm to 100 $\mu$m.

A preferred embodiment of the present invention provides an orally disintegrating porous film having therein a porosity of 10% to 35%, comprising a foaming agent of at least one selected from the group consisting of a nonionic surfactant, a cationic surfactant, an anionic surfactant, and an amphoteric surfactant, a foam stabilizer of at least one selected from the group consisting of pullulan, hydroxypropyl methylcellulose, polyvinylacetate, polyethylene oxide, xanthan gum, guar gum, locust bean gum, starch, starch derivatives, pectin, pectin hydrolyzates, alginic acid and alginic acid hydrolyzates, a plasticizer of at least one selected from the group consisting of glycerin, glycerol oleate, medium chain fatty acids, polyethylene glycol, propylene glycol, propylene glycol monocaprylate, propylene glycol dicaprylate, saccharides, sugar alcohols, and triacetin and a pharmacologically active ingredient.

In addition, the present invention provides a method of preparing an orally disintegrating porous film, which is suitable for a film formulation and has an improved drug dissolution rate.

More particularly, the present invention provides a method of preparing an orally disintegrating porous film, comprising (a) dissolving a foaming agent, a plasticizer and an excipient in a solvent to prepare a foamed solution; (b) adding a pharmacologically active ingredient to the solution and homogenizing the solution at 4,000 rpm to 15,000 rpm using a homogenizer to prepare a homogeneous solution; (c) adding a foam stabilizer to the homogenous solution and stirring the homogenous solution at 500 rpm to 1,500 rpm using a stirrer to prepare a foam-stabilized film solution; and (d) drying the film solution to mold a film.

The method of preparing the orally disintegrating porous film according to the present invention is very effective at increasing a drug dissolution rate by forming the micropores in the film while maintaining the properties of the film through a simple process.

The homogenizing in step (b) is performed at 4,000 rpm to 15,000 rpm, and the stirring in step (c) is carried out at 500 rpm to 1,500 rpm. These procedures are performed in a manner such that the foaming agent, such as a surfactant, is added, the production of foam is promoted at a high rpm, the foam stabilizer is added, and the stirring is conducted at a predetermined rpm, whereby the produced foam is maintained so as to be suitable for forming a film formulation, while the drug dissolution rate is also increased, unlike the typical preparation of an oral film formulation, in which defoaming is performed at a low rpm in order to completely remove generated foam. If the rates of the stirrer and the homogenizer are too low, the generation of foam is low and thus the drug dissolution rate may decrease. On the other hand, if the rates thereof are too high, the foam is generated in too large an amount, and thus, brittleness of the film may result.

If the viscosity of the film solution is too low, foam stability may decrease, thus making it impossible to prepare an orally disintegrating porous film. On the other hand, if the viscosity thereof is too high, it is impossible to mold a film. Hence, the viscosity of the film solution formed in step (c) of the method preferably falls in the range of 3,000 cps to 50,000 cps, which may be achieved by the addition of the foam stabilizer. More preferably, the film solution has a viscosity of 5,000 cps 20,000 cps. The viscosity of the film solution may be adjusted by varying the amounts of the film forming agent and the foam stabilizer.

In step (a), the solvent may include at least one selected from the group consisting of purified water, alcohol, alkyl acetate, dimethylformamide, dimethylsulfoxide, acetone, anisole, acetic acid, butyl methyl ether, ethyl ether, ethyl formate, formic acid, pentane, heptane, methylethylketone, and methyl isobutyl ketone.

In step (a), the foaming agent may include at least one selected from the group consisting of a surfactant, an animal-based foaming agent, a polymer foaming agent, and a mineral-based foaming agent, and the foam stabilizer used in step (b) may include at least one selected from the group consisting of pullulan, hydroxypropyl methylcellulose, polyvinylacetate, polyethylene oxide, xanthan gum, guar gum, locust bean gum, starch and starch derivatives, pectin and pectin hydrolyzates, alginic acid and alginic acid hydrolyzates.

In step (d), the drying temperature of film is preferably 50 to 160.

In the method of preparing the orally disintegrating porous film, a component, such as a flavor, oil, a coloring agent, a sweetener, an excipient, a disintegrant, an acidulant, etc., may be additionally added.

In step (d), the film may be molded through the following process.

Thus, a preferred embodiment of the present invention provides a method of preparing an orally disintegrating porous film having therein a porosity of 10% to 35%, comprising: (a) dissolving a foaming agent of at least one selected from the group consisting of a nonionic surfactant, a cationic surfactant, an anionic surfactant, and an amphoteric surfactant, a plasticizer of at least one selected from the group consisting of glycerin, glycerol oleate, medium chain fatty acid, polyethylene glycol, propylene glycol, propylene glycol monocaprylate, propylene glycol dicaprylate, saccharides, sugar alcohols, and triacetin, and an excipient in a solvent to prepare a foamed solution; (b) adding a pharmacologically active ingredient to the solution and homogenizing the solution at 4,000 rpm to 15,000 rpm using a homogenizer to prepare a homogenous solution; (c) adding a foam stabilizer of at least one selected from the group consisting of pullulan, hydroxypropyl methylcellulose, polyvinylacetate, polyethylene oxide, xanthan gum, guar gum, locust bean gum, starch, starch derivatives, pectin, pectin hydrolyzates, alginic acid, and alginic acid hydrolyzates to the homogenous solution, and stirring the homogenous solution at 500 rpm to 1,500 rpm using a stirrer to prepare a foam-stabilized film solution; and (d) drying the film solution to mold a film.

The prepared film solution is filtered and then placed in a molding machine so that a film is molded. As such, the temperature of the molding machine is preferably 50 to 150, and the solution is applied on a PET film and prepared in roll form using a belt dryer, thus obtaining a film.

The film thus molded is aged for about 1 to 10 days at a relative humidity of 50 to 70%, so that it has moisture content suitable for slitting or cutting. The moisture content preferably falls in the range of 6 to 12%. The aged roll is slit into small rolls, cut to an appropriate size, and placed in an aluminum package. The packaged aluminum product is further packaged in a small box, yielding the orally disintegrating porous film.

The pharmacologically active ingredient may include at least one selected from the group consisting of a therapeutic agent for diabetes, a therapeutic agent for insomnia, a therapeutic agent for urogenital organ, a therapeutic agent for obesity, an enzyme agent, an agent for peptic ulcer, an antitussive and an expectorant, a therapeutic agent for skin disease, an antiemetic agent, an anti-depressant, an antihistamine agent, an antipyretic analgesic anti-inflammatory drug, a hormonal agent, a therapeutic agent for circulatory organ, a therapeutic agent for digestive organ, a cardiovascular agent, a psychotropic agent, a therapeutic agent for erectile dysfunction, a therapeutic agent for osteoporosis, a therapeutic agent for arthritis, a therapeutic agent for epilepsy, a muscle relaxant, a brain function-improving agent, a therapeutic agent for schizophrenia, an immunosuppressant, an antibiotic agent, an anticancer drug, an anticancer treatment adjuvant, a vaccine agent, a mouth cleaning agent, a therapeutic agent for anemia, a therapeutic agent for constipation, a vitamin, a nutrient, a lactic acid bacteria agent, a multi-symptom cold medicine, an animal drug, and health functional food.

Also, the pharmacologically active ingredient may include at least one selected from the group consisting of L-menthol, nicotine, benzocaine, ascorbic acid, phentermine hydrochloride, caffeine, baclofen, memantine hydrochloride, selegiline hydrochloride, nitroglycerin, simethicone, diethyl propion, lamotrigine, pemirolast potassium, voglibose, mazindol, tizanidine hydrochloride, diclofenac, ramipril, ambroxol hydrochloride, alprazolam, lansoprazole, levonorgestrel, metoclopramide hydrochloride, famotidine, topiramate, tylpyridinium chloro, phendimetrazine tartrate, triazolam, omeprazole, ranitidine hydrochloride, torasemide, olopatadine hydrochloride, doxylamine succinate, dexamethasone, oxybutynin hydrochloride, citalopram bromide, tarafenacin, tamsulosin, sildenafil citrate, sildenafil, tadalafil, udenafil, mirodenafil hydrochloride, vardenafil hydrochloride, dapoxetine, donepezil hydrochloride, escitalopram oxalate, aripiprazole, atomoxetine hydrochloride, olanzapine, risperidone, meclizine, ramosetron hydrochloride, granisetron, ondansetron hydrochloride, ondansetron, aprepitant, montelukast, montelukast sodium, loratadine, desloratadine, chloropheniramine, phenylephrine hydrochloride, diphenhydramine hydrochloride, pseudoephedrine hydrochloride, levocetirizine hydrochloride, cetirizine hydrochloride, dextromethorphan bromide, ketoprofen, ibuprofen, acetaminophen, rizatriptan, zolmitriptan, sumatriptan, meloxicam, zolpidem, doxazosin mesylate, cimetidine, fentanyl, desmopressin, buprenorphine, oxycodone hydrochloride, naloxone hydrochloride, tianeptine, ebastine, solifenacin succinate, nicergoline, glimepiride, mosapride, loperamide hydrochloride, fesoterodine fumarate, fexofenadine hydrochloride, olmesartan medoxomil, amlodipine besylate, pharmaceutically acceptable salts or free bases thereof, and mixtures thereof.

In addition, the present invention provides an orally disintegrating porous film prepared by the above method.

The orally disintegrating porous film exhibits porosity but not brittleness, and may effectively manifest film properties and is also remarkably increased in the drug dissolution rate due to the presence of micropores therein. In particular, such a film is effective in the dissolution of poorly water soluble drugs.

Unless otherwise specified, the numerical values given in the specification should be understood to include their equivalent ranges.

A better understanding of the present invention is given through the following examples. However, such examples are merely set forth to illustrate, but are not to be construed as limiting the present invention.

**Mode for Invention**

**1. Comparative Examples 1 to 3 and Example 1**

**1. 1. Preparation of Comparative Example 1**

2.7 g of polysorbate 80, 2.7 g of glycerin, 2.7 g of polyethyleneglycol 600, 10 g of potassium dihydrogen phosphate, and 1 g of sodium hydroxide were dissolved in 150 g of purified water. The resulting solution was further added with 10 g of dextrin, 1.7 g of sucralose, 3 g of a flavor, 0.04 g of a coloring agent, and 25.16 g of an excipient, and then homogenized at 3,000 rpm using a homogenizer (IKAT25 digital UTRA-TURRAX). Thereafter, the resulting solution was added with 31 g of pullulan while stirring at 300 rpm using a stirrer (IKAEUROSTAR digital), yielding a foamed film solution.

The foamed coating solution thus stirred was allowed to stand overnight, and then molded into a film. Before the film molding process, the viscosity of the coating solution was measured to be 12,000 cps, and the density thereof was 0.9. The film was molded using a continuous film molding machine made by Ikeda (Japan), and was then subjected to slitting and cutting, thereby preparing a film.

**1.2. Preparation of Comparative Example 2**

A coating solution and then a film were prepared according to the same prescription and in the same manner as in Comparative Example 1, with the exception that the rates of the homogenizer and the stirrer were set to 6,000 rpm and 900 rpm, respectively. Before the film molding process, the coating solution had a viscosity of 12,000 cps and a density of 0.75.

### 1.3. Preparation of Comparative Example 3

A coating solution and then a film were prepared according to the same prescription and in the same manner as in Comparative Example 1, with the exception that 10 g of aripiprazole (an Abilify tablet), as a pharmaceutically active ingredient, and 15.16 g of an excipient were added. Before the film molding process, the coating solution had a viscosity of 12,000 cps and a density of 0.9.

### 1.4. Preparation of Example 1

A coating solution and then a film were prepared according to the same prescription and in the same manner as in Comparative Example 2, with the exception that 10 g of aripiprazole, as a pharmaceutically active ingredient, and 15.16 g of an excipient were added. Before the film molding process, the coating solution had a viscosity of 12,000 cps and a density of 0.76.

The formulations of Comparative Examples 1 to 3 and Example 1 and the preparation conditions of the film solutions are given in Tables 1 and 2 below.

[Table 1] Formulations

| Blending purpose | Standard | Maternal | Comparative Example 1 | Comparative Example 2 | Comparative Example 3 | Example 1 |
|---|---|---|---|---|---|---|
| | | | Added (g) | Added (g) | Added (g) | Added (g) |
| Foaming agent | USP/NF | Polysorbate80 | 2.7 | 2.7 | 2.7 | 2.7 |
| Plasticizer | USP | Glycerin | 2.7 | 2.7 | 2.7 | 2.7 |
| Plasticizer | USP/NF | Polyethylene glycol 60 | 2.7 | 2.7 | 2.7 | 2.7 |
| pH controller | USP/NF | potassium dihydrogen phosphate | 10 | 10 | 10 | 10 |
| pH controller | USP/NF | Sodium hydroxide | 1 | 1 | 1 | 1 |
| Excipient | USP/NF | Dextrin | 10 | 10 | 10 | 10 |
| Sweetener | USP/NF | Sucralose | 1.7 | 1.7 | 1.7 | 1.7 |
| Flavor | Appendix standard | Flavor | 3 | 3 | 3 | 3 |
| Coloring agent | USP/NF | Coloring agent | 0.04 | 0.04 | 0.04 | 0.04 |
| Excipient | USP/NF | Excipient | 25.16 | 25.16 | 15.16 | 15.16 |
| Main ingredient | Appendix standard | Aripiprazole | 0 | 0 | 10 | 10 |
| Foam stabilizer | USP/NF | Pullulan | 31 | 31 | 31 | 31 |

[Table 2] Preparation of Film Solution

| Item | Comparative Example 1 | Comparative Example 2 | Comparative Example 3 | Comparative Example 4 |
|---|---|---|---|---|
| Homogenizer rpm | 3,000 | 6,000 | 3,000 | 6,000 |

(continued)

| Item | Comparative Example 1 | Comparative Example 2 | Comparative Example 3 | Comparative Example 4 |
|---|---|---|---|---|
| Stirrer rpm | 300 | 900 | 300 | 900 |

### 1.5. Properties and Porosity of Films of Comparative

### Examples 1 to 3 and Example 1

The properties and porosity of the films of Comparative Examples 1 to 3 and Example 1 were measured, and whether the film was porous or not was observed using a microscope at 1200x magnification. Folding endurance was measured in such a manner that the orally disintegrating porous film of each of Comparative Examples 1 to 3 and Example 1 was allowed to stand overnight under conditions of 25°C and relative humidity 60 RH, after which the film was repeatedly subjected to folding in half and the number of times the film was folded was counted until the film broke.
The porosity of the prepared film was calculated using the following equation.

$$Relative\ Porosity = \frac{density\ of\ defoamed\ film - density\ of\ foamed\ film}{density\ of\ defoamed\ film} \times 100$$

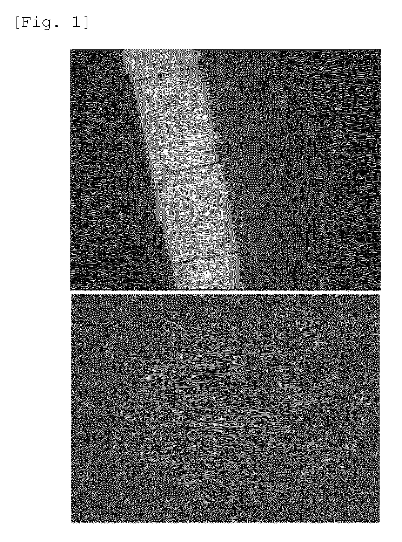

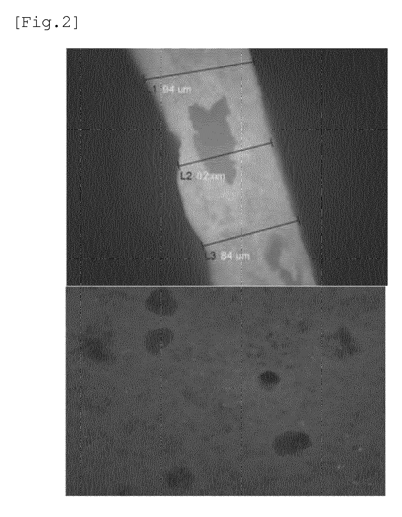

The results are shown in Table 3 below and FIGS. 1 and 2.

[Table 3]

| Item | Comparative Example 1 | Comparative Example 2 | Comparative Example 3 | Example 1 |
|---|---|---|---|---|
| Coating solution viscosity (cps) | 12,000 | 12,000 | 12,000 | 12,000 |
| Solution density | 0.9 | 0.75 | 0.9 | 0.76 |
| Porosity | 5% | 20% | 7% | 25% |
| Folding endurance (times) | 10 | 5 | 10 | 5 |

The film of Comparative Example 1 had good folding endurance but a low porosity of 5%. Although the film of Comparative Example 3 had good folding endurance, as seen in the microscopic results of FIG. 1, the film had a thickness of 63 μm, and pores were not clearly observed on the surface of the film, and the porosity thereof was merely 7%.
On the other hand, the films of Comparative Example 2 and Example 1 had a folding endurance value of 5, which was slightly lower than in Comparative Examples 1 and 3, but the properties thereof were maintained and the porosity thereof was 20% or more. When the rates of the homogenizer and the stirrer were increased to 6000 rpm and 900 rpm, respectively, foam was generated in the film solution, and the porosity of the resulting film was increased at least about 3 to 4 times, compared to Comparative Examples 1 and 3.
The cross-section of the film of Example 1 and the porosity thereof were observed using a microscope. The results are shown in FIG. 2.
As shown in FIG. 2, the thickness of the film of Example 1 was measured to be 90 μm due to the presence of pores, and the pores were observed on the surface of the film. When the generation of foam was promoted by increasing the rpm of the homogenizer and the rpm of the stirrer, a porous film formulation having micropores was confirmed to result. The film formulation of Example 1 according to the present invention exhibited good folding endurance despite the presence of pores and thus was confirmed to be usable as an orally disintegrating porous film formulation.

### 1.6. Comparison of Drug Dissolution Rates of Comparative

### Example 3 and Example 1

Since the dissolution of a drug is regarded as important in the orally disintegrating film formulation, the dissolution rate of the prepared film was measured. The dissolution rates of aripiprazole (an Abilify tablet), the non-porous aripiprazole orally disintegrating film of Comparative Example 3, and the porous aripiprazole orally disintegrating film of Example 1 were measured using the USP Abilify disintegrating tablet dissolution method.

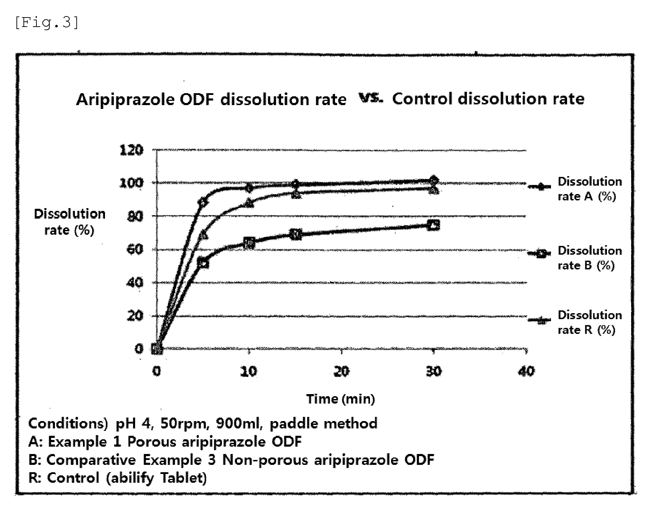

The results are shown in Table 4 below and FIG. 3.

[Table 4]

| Item | Control | C.Ex.3 | Ex.1 |
|---|---|---|---|
| Dissolution rate (10 min) (%) | 83 | 47 | 87 |
| Dissolution rate (30 min) (%) | 96 | 68 | 100 |

As is apparent from Table 4 and FIG. 3, the porous aripiprazole orally disintegrating film exhibited a high dissolution rate compared to the control, but the non-porous aripiprazole orally disintegrating film of Comparative Example 3 having low porosity manifested the dissolution rate corresponding to half the level of the control after 10 min.

As can be seen in the above results, the orally disintegrating film according to the present invention was prepared so as to possess folding endurance suitable for a film, as well as high porosity to thus exhibit a dissolution rate equal to or more than that of the disintegrating tablet. Hence, the film of the invention was confirmed to be useful as an excellent orally disintegrating film formulation.

**2. Examples 2 to 7 and Comparative Examples 4 to 9**

**2.1. Preparation of Examples 2 to 7 and Comparative**

**Examples 4 to 9**

The films of Examples 2 to 7 were prepared in the same manner as in Example 1, and the films of Comparative Examples 4 to 9 were prepared in the same manner as in Comparative Example 3, with the exception that a variety of active drugs were used based on the formulation of Table 5 below.

[Table 5]

| Blending purpose | Standard | Material | Example 2 Comparative example 4 | Example 3 Comparative example 5 | Example 4 Comparative example 6 | Example 5 Comparative example 7 | Example 6 Comparative example 8 | Example 7 Comparative example 9 |
|---|---|---|---|---|---|---|---|---|
| | | | Added (g) | Added (g) | Added (g) | Added (g) | Added (g) | Added (g) |
| Foaming agent | USP/NF | Polysorbate80 | 2.7 | 2.7 | 2.7 | 2.7 | 2.7 | 2.7 |
| Plasticizer | USP | Glycerin | 2.7 | 2.7 | 2.7 | 2.7 | 2.7 | 2.7 |
| Plasticizer | USP/NF | Polyethylene glycol 600 | 2.7 | 2.7 | 2.7 | 2.7 | 2.7 | 2.7 |
| pH controller | USP/NF | Potassium dihydrogen phosphate | 10 | 10 | 10 | 10 | 10 | 10 |
| pH controller | USP/NF | Sodium hydroxide | 1 | 1 | 1 | 1 | 1 | 1 |
| Excipient | USP/NF | Dextrin | 10 | 10 | 10 | 10 | 10 | 10 |
| Sweetener | USP/NF | Sucralose | 1.7 | 1.7 | 1.7 | 1.7 | 1.7 | 1.7 |
| Flavor | Appendix standard | Flavor | 3 | 3 | 3 | 3 | 3 | 3 |
| Coloring agent | USP/NF | Coloring agent | 0.04 | 0.04 | 0.04 | 0.04 | 0.04 | 0.04 |
| Excipient | USP/NF | Excipient | 15.16 | 15.16 | 15.16 | 15.16 | 15.16 | 15.16 |
| Main ingredient | | Ondansetron | 8 | - | - | - | - | - |
| | | Escitalopram oxalate | - | 10 | - | - | - | - |
| | | Tadalafil | - | - | 10 | - | - | - |
| | | Donepezil hydrochloride | - | - | - | 10 | - | - |
| | | Montelukast | - | - | - | - | 8 | - |
| | | Sillenafil citrate | - | - | - | - | - | 25 |

(continued)

| Blending purpose | Standard | Material | Example 2 Comparative example 4 | Example 3 Comparative example 5 | Example 4 Comparative example 6 | Example 5 Comparative example 7 | Example 6 Comparative example 8 | Example 7 Comparative example 9 |
|---|---|---|---|---|---|---|---|---|
| | | | Added (g) | Added (g) | Added (g) | Added (g) | Added (g) | Added (g) |
| Foam stabilizer | USP/NF | Pullulan | 31 | 31 | 31 | 31 | 31 | 31 |

[Table 6]

| | Example 2 | Example 3 | Example 4 | Example 5 | Example 6 | Example 7 |
|---|---|---|---|---|---|---|
| Porosity (%) | 21 | 20 | 19 | 20 | 23 | 22 |
| Folding endurance (times) | 5 | 4 | 6 | 5 | 4 | 6 |
| Item | Comparative example 4 | Comparative example 5 | Comparative example 6 | Comparative example 7 | Comparative example 8 | Comparative example 9 |
| Porosity (%) | 6 | 5 | 4 | 5 | 5 | 6 |
| Folding endurance (times) | 8 | 9 | 10 | 9 | 10 | 9 |

As is apparent from Table 6, the films of Examples 2 to 7 had folding endurance values of 4 to 6, which are slightly lower than in Comparative Examples, but the properties thereof were maintained and the porosity thereof was about 20%, which is much higher than Comparative Examples 4 to 9, which were characterized by about 5%. The films of Examples 2 to 7 according to the present invention were porous and had physical properties, such as folding endurance, suitable for use in films.

### 2.3. Drug Dissolution Rates of Examples 2 to 7 and

### Comparative Examples 4 to 9

The drug dissolution rates of the films of Examples 2 to 7 and Comparative Examples 4 to 9 were measured and compared. The dissolution rate of donepezil hydrochloride was measured using the USP Donepezil ODT dissolution method, and the dissolution rates of ondansetron, escitalopram oxalate, tadalafil, montelukast, and sildenafil citrate were measured according to the dissolution methods of respective USP tablets. The measured drug dissolution rates are shown in Table 7 below.

[Table 7]

| Item | Example 2 | Example 3 | Example 4 | Example 5 | Example 6 | Example 7 |
|---|---|---|---|---|---|---|
| Dissolution rate (10 min) (%) | 85 | 90 | 50 | 88 | 80 | 70 |
| Dissolution rate (30 min) (%) | 100 | 99 | 95 | 98 | 95 | 95 |
| Item | Comparative example 4 | Comparative example 5 | Comparative example 6 | Comparative example 7 | Comparative example 8 | Comparative example 9 |
| Dissolution rate (10 min) (%) | 70 | 75 | 30 | 70 | 60 | 50 |
| Dissolution rate (30 min) (%) | 95 | 95 | 90 | 89 | 92 | 90 |

As is apparent from Table 7, the porous films of Examples 2 to 7 were significantly improved in dissolution rates of all the drugs compared to Comparative Examples 4 to 9.

### 3. Examples 8 to 14

### 3.1 Preparation of Orally Disintegrating Porous Film of

### Examples 8 to 14

The films of Examples 8 to 14 were prepared in the same manner using the same device under the same construction as in Examples 1 to 7, with the exception that the porosity of the film was adjusted to 10%. Before the film molding process, the film solution had a viscosity of 12,00 cps, and the porosity of the prepared film was measured in the same manner as in Section 1.5 above. All of the porosities of the films of Examples 8 to 14 were about 10%.

### 3.2 Measurement of Folding Endurance and Drug Dissolution

### Rate of Examples 8 to 14

The folding endurance and drug dissolution rate of the films of Examples 8 to 14, having a porosity of 10%, were measured in the same manner as in Sections 1.5 and 1.6 above.
The dissolution rates of aripiprazole (an Abilify tablet) and the porous aripiprazole orally disintegrating film of Example 8 were measured using the USP Abilify disintegrating tablet dissolution method.
The results are shown in Table 8 below.

[Table 8]

| Item | Control | Example 8 |
|---|---|---|
| Porosity (%) | - | 10 |
| Dissolution rate (10 min) (%) | 83 | 85 |
| Dissolution rate (30 min) (%) | 96 | 97 |
| Folding endurance (times) | - | 9 |

As is apparent from Table 8, the orally disintegrating porous film of Example 8, having a porosity of 10%, had a folding endurance value of 9, which was regarded as appropriate for use as a film, and exhibited a drug dissolution rate equal to or more than that of the control. Hence, the film of this example was confirmed to be useful as an excellent orally disintegrating film formulation.
The folding endurance and drug dissolution rate of the films of Examples 9 to 14, having a porosity of 10%, were measured and respectively compared with those of Comparative Examples 4 to 9, having a porosity of less than 10%. The results are shown in Table 9 below.

[Table 9]

| Item | Comparative example 4 | Comparative example 5 | Comparative example 6 | Comparative example 7 | Comparative example 8 | Comparative example 9 |
|---|---|---|---|---|---|---|
| Porosity (%) | 6 | 5 | 4 | 5 | 5 | 6 |
| Dissolution rate (10 min) (%) | 70 | 75 | 30 | 70 | 60 | 50 |
| Dissolution rate (30 min) (%) | 95 | 95 | 90 | 89 | 92 | 90 |
| Folding endurance (times) | 8 | 9 | 10 | 9 | 10 | 9 |

(continued)

| Item | Example 9 | Example 10 | Example 11 | Example 12 | Example 13 | Example 14 |
|---|---|---|---|---|---|---|
| Porosity (%) | 10 | 10 | 10 | 10 | 10 | 10 |
| Dissolution rate (10 min) (%) | 84 | 88 | 51 | 85 | 61 | 65 |
| Dissolution rate (30 min) (%) | 97 | 96 | 95 | 98 | 96 | 96 |
| Folding endurance (times) | 8 | 9 | 9 | 8 | 9 | 9 |

As described above, the orally disintegrating porous films of Examples 9 to 14, having a porosity of 10%, had folding endurance values of 8 to 9, which were regarded as similar to those of the non-porous films of Comparative Examples 4 to 9, having a porosity of less than 10%, and exhibited drug dissolution rates superior to the respective corresponding comparative examples.

Therefore, the orally disintegrating porous film of Examples 8 to 14, having a porosity of 10%, was confirmed to be a film formulation suitable for the present invention, which is able to improve the drug dissolution rate while maintaining the properties of the film.

According to the present invention, the rapidly orally disintegrating porous film formulation, which is applicable to various drugs, can be prepared without additional processing, and can exhibit a higher dissolution rate than a non-porous oral film.

**Industrial Applicability**

According to the present invention, a rapidly disintegrating porous film formulation, which is applicable to various drugs, can be prepared without additional processing. The prepared rapidly disintegrating porous film formulation has a high dissolution rate compared to the non-porous film, and can thus be efficiently utilized in the preparation of a rapidly disintegrating film formulation having an improved dissolution rate.

**Claims**

1. An orally disintegrating porous film, comprising:

   a foaming agent, a foam stabilizer, a plasticizer, and a pharmacologically active ingredient.

2. The orally disintegrating porous film of claim 1, wherein the foaming agent is at least one selected from the group consisting of a surfactant, an animal-based foaming agent, a polymer foaming agent, and a mineral-based foaming agent.

3. The orally disintegrating porous film of claim 2, wherein the surfactant is at least one selected from the group consisting of a nonionic surfactant, a cationic surfactant, an anionic surfactant, and an amphoteric surfactant.

4. The orally disintegrating porous film of claim 3, wherein the surfactant is at least one selected from the group consisting of glycerin fatty acid ester, sucrose fatty acid ester, lecithin, enzyme-treated lecithin, polysorbate, sorbitan fatty acid ester, and sucrose fatty acid ester.

5. The orally disintegrating porous film of claim 1, wherein the foaming agent is in an amount of 0.5 wt% to 10 wt% based on a total weight of the orally disintegrating porous film.

6. The orally disintegrating porous film of claim 1, wherein the foam stabilizer is at least one selected from the group consisting of pullulan, hydroxypropyl methylcellulose, polyvinylacetate, polyethylene oxide, xanthan gum, guar gum, locust bean gum, starch and starch derivatives, pectin and pectin hydrolyzates, alginic acid and alginic acid hydrolyzates.

7. The orally disintegrating porous film of claim 1, wherein the foam stabilizer is in an amount of 20 wt% to 90 wt% based on a total weight of the orally disintegrating porous film.

8. The orally disintegrating porous film of claim 1, wherein the plasticizer comprises at least one selected from the group consisting of glycerin, glycerol oleate, medium chain fatty acid, polyethylene glycol, propylene glycol, propylene glycol monocaprylate, propylene glycol dicaprylate, saccharides, sugar alcohols, and triacetin.

9. The orally disintegrating porous film of claim 1, wherein the film has therein micropores having a size of 10 $\mu$m to 150 $\mu$m.

10. The orally disintegrating porous film of claim 1, wherein the film has therein a porosity of 8% to 40%.

11. The orally disintegrating porous film of claim 1, wherein the pharmacologically active ingredient is at least one selected from the group consisting of a therapeutic agent for diabetes, a therapeutic agent for insomnia, a therapeutic agent for urogenital organ, a therapeutic agent for obesity, an enzyme agent, an agent for peptic ulcer, an antitussive and an expectorant, a therapeutic agent for skin disease, an antiemetic agent, an anti-depressant, an antihistamine agent, an antipyretic analgesic anti-inflammatory drug, a hormonal agent, a therapeutic agent for circulatory organ, a therapeutic agent for digestive organ, a cardiovascular agent, a psychotropic agent, a therapeutic agent for erectile dysfunction, a therapeutic agent for osteoporosis, a therapeutic agent for arthritis, a therapeutic agent for epilepsy, a muscle relaxant, a brain function-improving agent, a therapeutic agent for schizophrenia, an immunosuppressant, an antibiotic agent, an anticancer drug, an anticancer treatment adjuvant, a vaccine agent, a mouth cleaning agent, a therapeutic agent for anemia, a therapeutic agent for constipation, a vitamin, a nutrient, a lactic acid bacteria agent, a multi-symptom cold medicine, an animal drug, and health functional food.

12. The orally disintegrating porous film of claim 1, wherein the pharmacologically active ingredient is at least one selected from the group consisting of L-menthol, nicotine, benzocaine, ascorbic acid, phentermine hydrochloride, caffeine, baclofen, memantine hydrochloride, selegiline hydrochloride, nitroglycerin, simethicone, diethyl propion, lamotrigine, pemirolast potassium, voglibose, mazindol, tizanidine hydrochloride, diclofenac, ramipril, ambroxol hydrochloride, alprazolam, lansoprazole, levonorgestrel, metoclopramide hydrochloride, famotidine, topiramate, tylpyridinium chloro, phendimetrazine tartrate, triazolam, omeprazole, ranitidine hydrochloride, torasemide, olopatadine hydrochloride, doxylamine succinate, dexamethasone, oxybutynin hydrochloride, citalopram bromide, tarafenacin, tamsulosin, sildenafil citrate, sildenafil, tadalafil, udenafil, mirodenafil hydrochloride, vardenafil hydrochloride, dapoxetine, donepezil hydrochloride, escitalopram oxalate, aripiprazole, atomoxetine hydrochloride, olanzapine, risperidone, meclizine, ramosetron hydrochloride, granisetron, ondansetron hydrochloride, ondansetron, aprepitant, montelukast, montelukast sodium, loratadine, desloratadine, chlorpheniramine, phenylephrine hydrochloride, diphenhydramine hydrochloride, pseudoephedrine hydrochloride, levocetirizine hydrochloride, cetirizine hydrochloride, dextromethorphan bromide, ketoprofen, ibuprofen, acetaminophen, rizatriptan, zolmitriptan, sumatriptan, meloxicam, zolpidem, doxazosin mesylate, cimetidine, fentanyl, desmopressin, buprenorphine, oxycodone hydrochloride, naloxone hydrochloride, tianeptine, ebastine, solifenacin succinate, nicergoline, glimepiride, mosapride, loperamide hydrochloride, fesoterodine fumarate, fexofenadine hydrochloride, olmesartan medoxomil, amlodipine besylate, pharmaceutically acceptable salts or free bases thereof, and mixtures thereof.

13. A method of preparing an orally disintegrating porous film, comprising:

(a) dissolving a foaming agent, a plasticizer and an excipient in a solvent to prepare a foamed solution;
(b) adding a pharmacologically active ingredient to the solution and homogenizing the solution at 4,000 rpm to 15,000 rpm using a homogenizer to prepare a homogenous solution;
(c) adding a foam stabilizer to the homogenous solution and stirring the homogenous solution at 500 rpm to 1,500 rpm using a stirrer to prepare a foam-stabilized film solution; and
(d) drying the film solution to mold a film.

14. The method of claim 13, wherein the film solution has a viscosity of 3,000 cps to 50,000 cps.

15. The method of claim 13, wherein the drying temperature of the film solution in (d) is 50 to 160°C.

**16.** The method of claim 13, wherein the foaming agent is at least one selected from the group consisting of a surfactant, an animal-based foaming agent, a polymer foaming agent, and a mineral-based foaming agent.

**17.** The method of claim 13, wherein the foam stabilizer is at least one selected from the group consisting of pullulan, hydroxypropyl methylcellulose, polyvinylacetate, polyethylene oxide, xanthan gum, guar gum, locust bean gum, starch and starch derivatives, pectin and pectin hydrolyzates, alginic acid and alginic acid hydrolyzates.

**18.** The method of claim 13, wherein the pharmacologically active ingredient is at least one selected from the group consisting of a therapeutic agent for diabetes, a therapeutic agent for insomnia, a therapeutic agent for urogenital organ, a therapeutic agent for obesity, an enzyme agent, an agent for peptic ulcer, an antitussive and an expectorant, a therapeutic agent for skin disease, an antiemetic agent, an anti-depressant, an antihistamine agent, an antipyretic analgesic anti-inflammatory drug, a hormonal agent, a therapeutic agent for circulatory organ, a therapeutic agent for digestive organ, a cardiovascular agent, a psychotropic agent, a therapeutic agent for erectile dysfunction, a therapeutic agent for osteoporosis, a therapeutic agent for arthritis, a therapeutic agent for epilepsy, a muscle relaxant, a brain function-improving agent, a therapeutic agent for schizophrenia, an immunosuppressant, an antibiotic agent, an anticancer drug, an anticancer treatment adjuvant, a vaccine agent, a mouth cleaning agent, a therapeutic agent for anemia, a therapeutic agent for constipation, a vitamin, a nutrient, a lactic acid bacteria agent, a multi-symptom cold medicine, an animal drug, and health functional food.

**19.** The method of claim 13, wherein the pharmacologically active ingredient is at least one selected from the group consisting of L-menthol, nicotine, benzocaine, ascorbic acid, phentermine hydrochloride, caffeine, baclofen, memantine hydrochloride, selegiline hydrochloride, nitroglycerin, simethicone, diethyl propion, lamotrigine, pemirolast potassium, voglibose, mazindol, tizanidine hydrochloride, diclofenac, ramipril, ambroxol hydrochloride, alprazolam, lansoprazole, levonorgestrel, metoclopramide hydrochloride, famotidine, topiramate, tylpyridinium chloro, phendimetrazine tartrate, triazolam, omeprazole, ranitidine hydrochloride, torasemide, olopatadine hydrochloride, doxylamine succinate, dexamethasone, oxybutynin hydrochloride, citalopram bromide, tarafenacin, tamsulosin, sildenafil citrate, sildenafil, tadalafil, udenafil, mirodenafil hydrochloride, vardenafil hydrochloride, dapoxetine, donepezil hydrochloride, escitalopram oxalate, aripiprazole, atomoxetine hydrochloride, olanzapine, risperidone, meclizine, ramosetron hydrochloride, granisetron, ondansetron hydrochloride, ondansetron, aprepitant, montelukast, montelukast sodium, loratadine, desloratadine, chloropheniramine, phenylephrine hydrochloride, diphenhydramine hydrochloride, pseudoephedrine hydrochloride, levocetirizine hydrochloride, cetirizine hydrochloride, dextromethorphan bromide, ketoprofen, ibuprofen, acetaminophen, rizatriptan, zolmitriptan, sumatriptan, meloxicam, zolpidem, doxazosin mesylate, cimetidine, fentanyl, desmopressin, buprenorphine, oxycodone hydrochloride, naloxone hydrochloride, tianeptine, ebastine, solifenacin succinate, nicergoline, glimepiride, mosapride, loperamide hydrochloride, fesoterodine fumarate, fexofenadine hydrochloride, olmesartan medoxomil, amlodipine besylate, pharmaceutically acceptable salts or free bases thereof, and mixtures thereof.

**20.** An orally disintegrating porous film, prepared by the method of any one of claims 13 to 19.

[Fig. 1]

[Fig.2]

[Fig.3]

**Aripiprazole ODF dissolution rate vs. Control dissolution rate**

Conditions) pH 4, 50rpm, 900ml, paddle method
A: Example 1 Porous aripiprazole ODF
B: Comparative Example 3 Non-porous aripiprazole ODF
R: Control (abilify Tablet)

## INTERNATIONAL SEARCH REPORT

| | International application No. |
| --- | --- |
| | **PCT/KR2014/010293** |

### A. CLASSIFICATION OF SUBJECT MATTER

*A61K 9/70(2006.01)i, A61P 3/10(2006.01)i, A61P 3/04(2006.01)i*

According to International Patent Classification (IPC) or to both national classification and IPC

### B. FIELDS SEARCHED

Minimum documentation searched (classification system followed by classification symbols)
A61K 9/70; A61K 9/14; A61K 31/235; A61K 9/44; A61K 9/16; A61K 31/192; A61K 31/74; A61K 31/519; A61P 3/10; A61P 3/04

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: film, surfactant, porous, stirring, flurane

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2013-0100037 A (SEOUL PHARMA. CO., LTD.) 09 September 2013 See paragraphs [0077-0079], examples 1-27. | 1-12 |
| A | | 13-20 |
| X | KR 10-0627199 B1 (LAVIPHARM LABORATORIES, INC.) 22 September 2006 See pages 5-7, table of the examples 1, 3, 5, 7. | 1-12 |
| A | | 13-20 |
| X | US 2011-0086070 A1 (TALWAR,M. et al.) 14 April 2011 See examples 6-8. | 1-12 |
| A | | 13-20 |
| X | US 7067116 B1 (BESS,W.S. et al.) 27 June 2006 See the examples. | 1-12 |
| A | | 13-20 |
| A | KR 10-1320058 B1 (CHUNG ANG UNIVERSITY INDUSTRY ACADEMIC COOPERATION FOUNDATION) 18 October 2013 See the entire main text. | 1-20 |
| A | KR 10-2011-0044752 A (BAYER PHARMA AKTIENGESELLSCHAFT) 29 April 2011 See the examples. | 1-20 |

| ☐ Further documents are listed in the continuation of Box C. | ☒ See patent family annex. |
| --- | --- |

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| 17 DECEMBER 2014 (17.12.2014) | **18 DECEMBER 2014 (18.12.2014)** |

| Name and mailing address of the ISA/KR <br> Korean Intellectual Property Office <br> Government Complex-Daejeon, 189 Seonsa-ro, Daejeon 302-701, Republic of Korea <br> Facsimile No. 82-42-472-7140 | Authorized officer <br><br><br> Telephone No. |
| --- | --- |

Form PCT/ISA/210 (second sheet) (July 2009)

INTERNATIONAL SEARCH REPORT
Information on patent family members

International application No.

**PCT/KR2014/010293**

| Patent document cited in search report | Publication date | Patent family member | Publication date |
|---|---|---|---|
| KR 10-2013-0100037 A | 09/09/2013 | KR 10-1440808 B1 | 04/11/2014 |
| | | WO 2013-129889 A2 | 06/09/2013 |
| | | WO 2013-129889 A3 | 06/09/2013 |
| | | WO 2013-129889 A9 | 06/09/2013 |
| KR 10-0627199 B1 | 22/09/2006 | AU 2000-22226 A1 | 07/08/2000 |
| | | AU 2000-22226 B2 | 16/09/2004 |
| | | CA 2358524 A1 | 27/07/2000 |
| | | CN 100389755 C0 | 28/05/2008 |
| | | CN 1354656 A0 | 19/06/2002 |
| | | EP 1143940 A2 | 17/10/2001 |
| | | EP 1143940 B1 | 19/04/2006 |
| | | JP 2002-535269A | 22/10/2002 |
| | | JP 2012-072166A | 12/04/2012 |
| | | US 6552024 B1 | 22/04/2003 |
| | | WO 00-42992 A2 | 27/07/2000 |
| | | WO 00-42992 A3 | 19/10/2000 |
| US 2011-0086070 A1 | 14/04/2011 | EP 2268268 A2 | 05/01/2011 |
| | | WO 2009-118589 A2 | 01/10/2009 |
| | | WO 2009-118589 A3 | 14/01/2010 |
| US 7067116 B1 | 27/06/2006 | AU 2001-229720 B2 | 02/02/2006 |
| | | AU 2001-29720 A1 | 03/10/2001 |
| | | AU 2006-201888 A1 | 25/05/2006 |
| | | AU 2006-201888 B2 | 11/06/2009 |
| | | AU 2006-201899 A1 | 25/05/2006 |
| | | AU 2972001 A | 03/10/2001 |
| | | CA 2402988 A1 | 27/09/2001 |
| | | CA 2402988 C | 14/07/2009 |
| | | CN 100525835 C | 12/08/2009 |
| | | CN 1181814 C0 | 29/12/2004 |
| | | CN 1419441 A0 | 21/05/2003 |
| | | CN 1651092 A | 10/08/2005 |
| | | CN 1651092 C0 | 10/08/2005 |
| | | DE 60119298 D1 | 08/06/2006 |
| | | DE 60119298 T2 | 31/08/2006 |
| | | DK 1267829 T3 | 11/09/2006 |
| | | EP 1267829 A1 | 02/01/2003 |
| | | EP 1267829 B1 | 03/05/2006 |
| | | EP 1674078 A2 | 28/06/2006 |
| | | EP 1674078 A3 | 25/07/2007 |
| | | JP 04-145048B2 | 03/09/2008 |
| | | JP 2003-527410 T | 16/09/2003 |
| | | JP 2003-527410A | 16/09/2003 |
| | | KR 10-2002-0084233 A | 04/11/2002 |
| | | US 2006-0204559 A1 | 14/09/2006 |
| | | US 7648712 B2 | 19/01/2010 |
| | | WO 01-70194 A1 | 27/09/2001 |

Form PCT/ISA/210 (patent family annex) (July 2009)

INTERNATIONAL SEARCH REPORT
Information on patent family members

| International application No. |
| --- |
| **PCT/KR2014/010293** |

| Patent document cited in search report | Publication date | Patent family member | Publication date |
| --- | --- | --- | --- |
| | | WO 01-70194A1 | 27/09/2001 |
| KR 10-1320058 B1 | 18/10/2013 | KR 10-2013-0003511 A | 09/01/2013 |
| KR 10-2011-0044752 A | 29/04/2011 | AU 2009-214193 A1 | 20/08/2009 |
| | | AU 2009-214307 A1 | 20/08/2009 |
| | | AU 2009-279053 A1 | 11/02/2010 |
| | | CA 2714340 A1 | 20/08/2009 |
| | | CA 2714598 A1 | 20/08/2009 |
| | | CA 2732211 A1 | 11/02/2010 |
| | | CA 2744127 A1 | 27/05/2010 |
| | | CN101945646 A | 12/01/2011 |
| | | CN102006857 A | 06/04/2011 |
| | | CN102006857 B | 26/06/2013 |
| | | CN102119021 A | 06/07/2011 |
| | | CN102215818 A | 12/10/2011 |
| | | EP 2249803 A2 | 17/11/2010 |
| | | EP 2252261 A2 | 24/11/2010 |
| | | EP 2331067 A1 | 15/06/2011 |
| | | EP 2358351 A1 | 24/08/2011 |
| | | JP 2011-511816A | 14/04/2011 |
| | | JP 2011-511823A | 14/04/2011 |
| | | JP 2011-530499A | 22/12/2011 |
| | | JP 2012-509286A | 19/04/2012 |
| | | KR 10-2010-0117603 A | 03/11/2010 |
| | | KR 10-2010-0125309 A | 30/11/2010 |
| | | KR 10-2011-0097779 A | 31/08/2011 |
| | | US 2011-0052699 A1 | 03/03/2011 |
| | | US 2011-0097405 A1 | 28/04/2011 |
| | | US 2011-0293720 A1 | 01/12/2011 |
| | | US 2012-0282340 A1 | 08/11/2012 |
| | | WO 2009-100871 A2 | 20/08/2009 |
| | | WO 2009-100871 A3 | 23/12/2009 |
| | | WO 2009-101021 A2 | 20/08/2009 |
| | | WO 2010-015713 A1 | 11/02/2010 |
| | | WO 2010-057594 A1 | 27/05/2010 |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120149713 A
- US 7425292 B
- KR 101303479
- KR 101188594
- KR 1020130003511
- KR 100937625